# EUROPEAN PATENT APPLICATION

(11) **EP 4 011 415 A1**
(43) Date of publication of application: **15.06.2022**
(21) Application number: 20213764.2
(22) Date of filing: 14.12.2020
(51) Int. Cl.: A61M 1/06

(54) **BREAST PUMP**

(71) Applicant: Koninklijke Philips N.V., 5656 AG Eindhoven (NL)
(72) Inventor: CLAASSEN, Coen Petrus Martinus, 5656 AE Eindhoven (NL); VAN VELDHUIZEN, Gijsbert, 5656 AE Eindhoven (NL); DOBRUSSKIN, Christoph, 5656 AE Eindhoven (NL)
(74) Representative: Philips Intellectual Property & Standards

(57) **Abstract**

A breast pump comprises an expression system. Audible and/or tactile feedback is generated for a user of the breast pump by controlling the expression system.

## Description

### FIELD OF THE INVENTION

This invention relates to breast pumps.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted. In full function designs, the breast pump has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

The vacuum pump is able to achieve a set vacuum level, which can be held at a desired level by shutting off the pump, and can then be released by opening a (solenoid) valve. In this way, a cyclic pressure waveform can be created.

Wearable breast pumps are characterized in that they can be fully worn on the user's body, allowing the user to move around freely. Some of these are small enough to fit into a user's bra. A motor, battery and milk container are for example integrated to form a single unit.

It is very common to have some kind of interface to provide feedback to the user, such as a tactile or audible feedback when control buttons are pressed, or feedback for notifying the user that device the expression cycle is finished, or for an empty battery alert etc.. This user feedback is traditionally delivered by means of sound, light, haptic feedback, mobile phone notification or other methods.

However, these solutions all need a dedicated feedback system, which increases product cost and size.

### SUMMARY OF THE INVENTION

The invention is defined by the claims.

According to examples in accordance with an aspect of the invention, there is provided a breast pump comprising:
an expression system for expressing milk;
a drive arrangement for controlling the expression process; and
a controller for controlling the drive arrangement,
wherein the controller is adapted to control the drive arrangement thereby to generate audible and/or tactile feedback to a user of the breast pump.

The expression for example comprises at least a unit for interfacing with the breast of a user, such as a breast funnel or breast shield. It may additionally include a milk collecting receptacle and other components which are located in the vicinity of the breast.

This breast pump uses control of the drive arrangement (which is for controlling the expression process) to provide feedback to the user. The feedback provides information to the user relating to the operation of the breast pump, for example information about the status of the breast pumping session, or confirmatory feedback in response to input commands provided by the user. As long as the feedback differs from the sound or feel during use of the breast pump to perform expression (or stimulation), it can provide information content to the user. The feedback may be provided during times of interruption of the normal operation of the breast pump if the feedback is needed is during breast pumping, or it may be before the start of the expression or after the end of the expression.

The expression system for example comprises a vacuum chamber and a pump arrangement, and the drive arrangement is for driving the pump arrangement to control an under-pressure in the vacuum chamber. The expression system for example further comprises a valve for relating the under-pressure from the vacuum chamber and the drive arrangement is for controlling the valve.

This arrangement is able to generate cyclic pressure waveforms, such as a stimulation waveform and an expression waveform.

The breast pump may thus use control of the release valve and/or the pump arrangement (e.g. the pump motor) to provide feedback to the user.

The feedback uses existing components which are needed for the other functions of the breast pump, e.g. a controller, valve and impeller and pump motor in this particular example. Thus, no additional components are required.

The controller may be adapted to a generate respective feedback signal for one or more of the following indications:
a user has pressed an input button of the breast pump;
a user has set the breast pump to a maximum setting.

The feedback may instead relate to a status of the breast pump and its operation. For example a respective feedback signal may be generated for one or more of the following indications:
an expression cycle has ended;
a battery of the breast pump is at or approaching depletion;
a milk container of the breast pump is full;
a remaining time for the expression cycle or other time indication;
a state of readiness;
a malfunction; or
a leak detection.

These feedback options relate to providing status reports to the user.

The controller may be adapted to generate a cyclic pressure waveform in use of the breast pump, and the controller is adapted to generate a different pressure waveform thereby to implement an audible and/or tactile feedback signal.

The cyclic pressure waveform then generates a different sound and/or feel to a sound and/or feel of the audible and/or tactile feedback signal. Thus, the sound and/or feel of the feedback signal is different to the normal sound of the breast pump cyclic operation.

The controller may be adapted to generate at least one feedback signal by operating the pump arrangement with the valve open. This provides a sound (e.g. a hissing sound) not present during normal operation, and creates no or little vacuum in the chamber.

The controller may be adapted to generate at least one feedback signal by opening the valve without an under-pressure in the chamber. This again provides a sound (e.g. a click sound) not present during normal operation.

The controller may be adapted to generate the at least one feedback signal by generating a sequence of pulses of operation of the pump arrangement and/or valve. The timing and overall duration of the pulses may then encode different feedback messages. The level of vacuum allowed to build in the chamber may also be used to generate different sounds when the vacuum is released.

Thus, the controller may be adapted to generate a selected one of a set of feedback signals for different feedback messages, each feedback signal comprising a generation of a different sequence of pulses of operation of the driver arrangement, e.g. the motor and/or valve.

The breast pump may further comprise a milk collection vessel. It may be a wearable breast pump, for example for fitting in a feeding bra. A "wearable" breast pump may be considered to be one for which the expression kit, drive arrangement, and the milk collection vessel are mounted locally at the breast.

The invention also provides a method of operating a breast pump, the breast pump comprising an expression system for expressing milk and a drive arrangement for controlling the expression process, wherein the method comprises:
controlling the driver arrangement to generate audible and/or tactile feedback to a user of the breast pump.

This method uses the drive arrangement to control existing parts of the expression system to function as a tactile and/or audible feedback system.

The method may comprise generating a respective feedback signal for one or more of the following indications:
a user has pressed an input button of the breast pump;
a battery of the breast pump is at or approaching depletion;
a milk container of the breast pump is full;
a remaining time for the expression cycle; or
an expression cycle has ended;
a state of readiness;
a malfunction;
a time indication; or
a leak detection.

The expression system may comprise a vacuum chamber, a pump arrangement and a valve for releasing the under-pressure from the vacuum chamber and the drive arrangement is for controlling the pump arrangement and valve.

The method may then comprise generating at least one feedback signal by:
operating the pump arrangement with the valve open;
opening the valve without an under-pressure in the chamber; or generating a sequence of pulses of operation of the motor and/or valve.

The invention also provides a computer program comprising computer program code means which is adapted, when said program is run on a processor, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known breast pump, which may be controlled in accordance with the invention;
Fig. 2 shows a wearable breast pump; and
Fig. 3 shows some examples of sound profile that may be created as feedback signals.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump which comprises an expression system. Audible and/or tactile feedback is generated for a user of the breast pump by controlling the expression system.

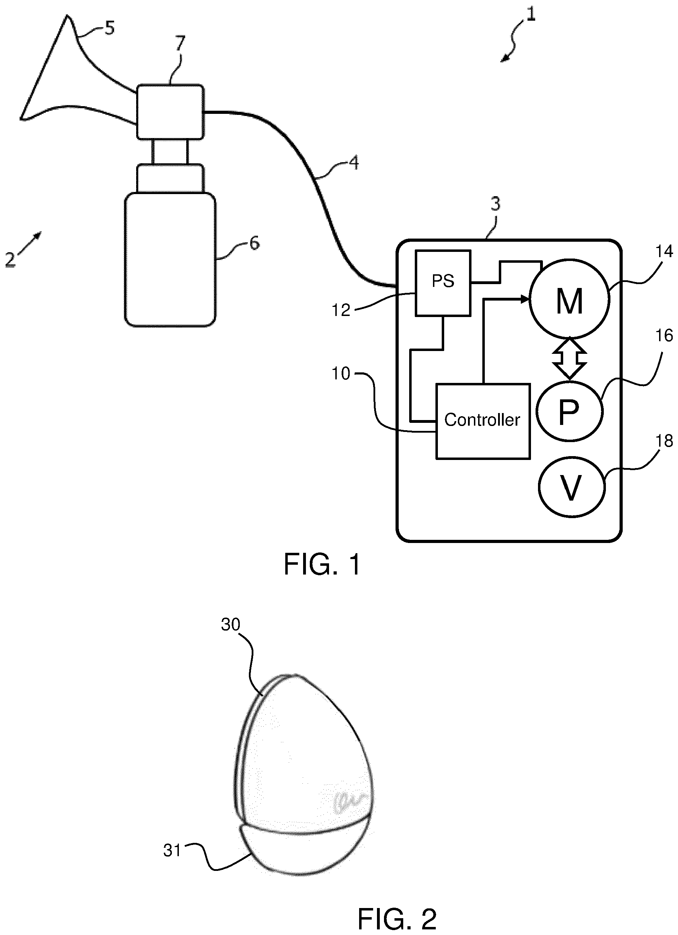

Fig. 1 shows a known breast pump system 1, comprising an expression unit 2 and a drive arrangement for controlling the expression function of the breast pump.

In the example shown, the drive arrangement comprises a pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 for receiving a breast of a user and a receptacle 6 for collecting the expressed milk. The funnel 5 and the receptacle 6 are connected to the main body 7. The main body 7 comprises a vacuum chamber. A flexible membrane or diaphragm is located in the vacuum chamber. The membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump applies a vacuum to the membrane located in the main body 7 so that it deforms. The membrane deforms to create a vacuum in the funnel 5 which in turns applies a vacuum to the breast which enables milk to be expressed. Although the breast pump system 1 is described as comprising a membrane such that the vacuum is applied indirectly to the breast, it should be understood that in an alternative embodiment, the vacuum is applied directly to the breast of a user. In this case, the breast pump system does not comprise a membrane and the vacuum created by the vacuum pump is applied directly to the breast.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection vessel. This enables breast pumping to be performed more discretely.

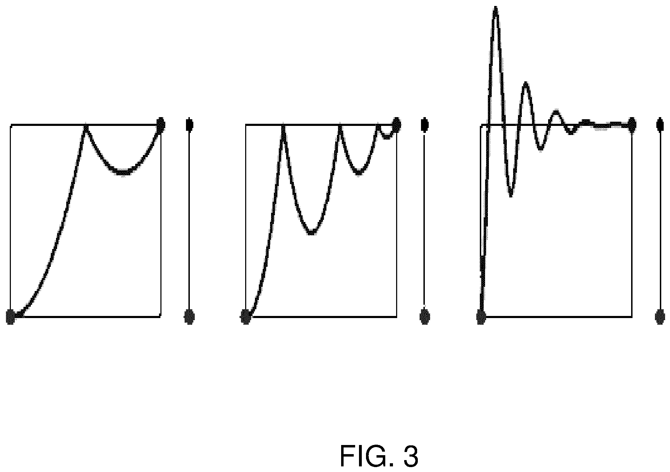

Fig. 3 shows a wearable breast pump, comprising an expression kit 30 attached to a respective milk collection vessel 31.

In both general types of design, the expression kit may comprise a milk sensor for sensing milk flow and for measuring flow volume. Also, different pressure waveforms may for example be selected by a user, for example different intensity levels, depending on the level of comfort of the mother to those different intensity levels.

Different modes and settings have different cyclic frequency and different maximum under-pressure (i.e. vacuum) levels.

An expression mode for example cycles between a baseline vacuum "Baseline Vac" and a maximum vacuum (i.e. a maximum under-pressure) "Max Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline_Vac = atmospheric pressure
Max_Vac = 300 mbar (30 kPa) below atmospheric pressure
TTV = 0.85 s
DI = 0.3 s
TTA = 0.05 s
DO = 0.35 s

This gives a total cycle time T_cycle of 1.55s.

These values are simply by way of example. The maximum under-pressure may be higher, for example 350 mbar (35 kPa) and the the durations may differ. The cycle time is generally of the order of 1 to 2 seconds.

A stimulation mode for example has a faster frequency hence with a shorter cycle time of around 0.5 seconds, and a lower vacuum, for example a maximum under-pressure level of 150 mbar (15 kPa).

In an expression kit with both stimulation mode and expression modes, the stimulation mode for example takes placing during an initial period of the breast pumping session (e.g. 50 seconds) and the expression mode takes place during the remainder of the breast pumping session. There may also be different settings of expression mode, again with different characteristics.

A controller may also implement a positioning mode. This may comprise intermittent application of a small under-pressure to allow the user to position the breast shield while observing the correct alignment. A controller may also implement a massage mode.

The various modes and settings will result in different sounds generated by the breast pump. In accordance with the invention, the control of the drive arrangement, for example the valve and/or the motor of the pump arrangement for the system example above, is also used to generate additional user-recognizable sounds or tactile feedback (e.g. characteristic vibrations).

The feedback provides information to the user relating to the operation of the breast pump, for example information about the status of the breast pumping session, or confirmatory feedback in response to input commands provided by the user. As long as the feedback differs from the sound and/or feel during use of the breast pump in its various modes, it can provide information content to the user.

The operation of the motor and valve will of course influence the breast pumping function. Thus, the feedback may be provided during a short time of interruption of the normal cyclic operation of the breast pump, for example missing one or a small number of cycles, if the feedback is needed during breast pumping. The feedback may instead be before the start of the expression cycles or after the end of the expression cycles.

The feedback may be superposed over a normal cyclic operation of the breast pump by providing a modulation which still allows the cyclic control to continue but provides a recognizable change in sound and/or feel.

Another type of feedback may be the interruption of the normal sound and feel, i.e. a period of silence (and hence no vibration). The duration or pattern of such a period or periods of silence may then convey feedback information.

The feedback may be used for various purposes. User inputs, such as pressing an input button.

Other feedback may relate to status information about the general status of the breast pump and its operation.

Status reports to the user for example comprise notifications indicating:
an expression cycle has ended;
a battery of the breast pump is at or approaching depletion;
a milk container of the breast pump is full;
a remaining time for the expression cycle or other time indication;
a state of readiness;
a malfunction; or
a leak detection.

The recognizable sounds may be generated by operating the breast pump differently to during normal use. One example is to operate the motor with the valve open. This provides a sound (e.g. a hissing sound) not present during normal operation. Another example is to open the valve without an under-pressure in the chamber. This again provides a sound (e.g. a click sound) not present during normal operation.

Fig. 3 shows three possible feedback intensity profiles of amplitude versus time. The feedback may be audible or tactile as explained above.

Fig. 3 shows in schematic form that the feedback profile may have a desired pulsing frequency as well as a desired amplitude characteristic, either with sudden changes in amplitude (e.g. clicks or sharp vibrations) or gradual amplitude changes.

Thus, the feedback signal in these examples comprises a sequence of feedback pulses, induced by suitable control of the operation of the drive arrangement, e.g. the pump motor and/or valve. The timing, amplitude response and the overall duration of the pulses may then encode different feedback messages.

Tactile signals may also be distinguished from each other by a similar approach, with different vibration frequency, vibration amplitude and vibration duration.

The example above shows a drive arrangement which comprises a pump arrangement (pump motor and impeller) and a release valve. However, other drive arrangements are possible.

For example an arrangement may be used to create a base vacuum which is modulated around a preferred level. Such a base vacuum can be created manually or mechanically by e.g. a servo-actuated bellows. Vacuum modulation can be created for example lowering the air pressure in a defined volume by a pump or increasing the vacuum volume by a mechanical actuation.

As another example, an arrangement can be made based on a plunger-based pumping system creating either a peak vacuum which is cyclically released by a valve or a base vacuum which is modulated by e.g. the same plunger pump.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump comprising:
an expression system for expressing milk;
a drive arrangement for controlling the expression process; and
a controller (10) for controlling the drive arrangement,
wherein the controller is adapted to control the drive arrangement thereby to generate audible and/or tactile feedback to a user of the breast pump.

2. The breast pump of claim 1, wherein the controller (10) is adapted to a generate respective feedback signal for one or more of the following indications:
a user has pressed an input button of the breast pump;
a battery of the breast pump is at or approaching depletion;
a milk container of the breast pump is full;
a remaining time for the expression cycle or other time indication;
an expression cycle has ended;
a state of readiness;
a malfunction; or
a leak detection.

3. The breast pump of claim 1 or 2, wherein the expression system comprises a vacuum chamber and a pump arrangement (14, 16), and the drive arrangement is for driving the pump arrangement to control an under-pressure in the vacuum chamber.

4. The breast pump of claim 3, wherein the expression system comprises a valve (18) for releasing the under-pressure from the vacuum chamber and the drive arrangement is for controlling the valve.

5. The breast pump of claim 4, wherein the controller (10) is adapted to generate a cyclic pressure waveform in use of the breast pump, and the controller is adapted to generate a different pressure waveform thereby to implement an audible and/or tactile feedback signal.

6. The breast pump of claim 4 or 5, wherein the controller (10) is adapted to generate at least one feedback signal by:
operating the pump arrangement with the valve open; or
opening the valve without an under-pressure in the chamber.

7. The breast pump of any one of claims 3 to 6, wherein the controller (10) is adapted to generate the at least one feedback signal by generating a sequence of pulses of operation of the pump arrangement and/or valve.

8. The breast pump of claim 7, wherein the controller (10) is adapted to generate a selected one of a set of feedback signals for different feedback messages, each feedback signal comprising a generation of a different sequence of pulses of operation of the pump arrangement and/or valve.

9. The breast pump of any one of claims 1 to 8, wherein the controller (10) is adapted to generate at least one feedback signal by providing a pause in the control of the drive arrangement.

10. The breast pump of any one of claims 1 to 9, further comprising a milk collection vessel (31) and wherein the breast pump comprises a wearable breast pump (30,31).

11. A method of operating a breast pump, the breast pump comprising an expression system for expressing milk and a drive arrangement for controlling the expression process, wherein the method comprises:
controlling the driver arrangement to generate audible and/or tactile feedback to a user of the breast pump.

12. The method of claim 11, comprising generating a respective feedback signal for one or more of the following indications:
a user has pressed an input button of the breast pump;
a battery of the breast pump is at or approaching depletion;
a milk container of the breast pump is full;
a remaining time for the expression cycle or other time indication;
an expression cycle has ended;
a state of readiness;
a malfunction; or
a leak detection.

13. The method claim 11 or 12, wherein the expression system comprises a vacuum chamber, a pump arrangement (14, 16) and a valve (18) for releasing the under-pressure from the vacuum chamber and the drive arrangement is for controlling the pump arrangement and valve.

14. The method of claim 13, comprising generating at least one feedback signal by:
operating the pump arrangement with the valve open;
opening the valve without an under-pressure in the chamber;
providing a pause in the control of the drive arrangement; or
generating a sequence of pulses of operation of the pump arrangement and/or valve.

15. A computer program comprising computer program code means which is adapted, when said program is run on a processor, to implement the method of any one of claims 11 to 14.
